# EUROPEAN PATENT SPECIFICATION

(11) **EP 1 732 558 B1**
(45) Date of publication and mention of the grant of the patent: **29.09.2010**
(21) Application number: 05729786.3
(22) Date of filing: 23.03.2005
(51) Int. Cl.: A61K 31/496, A61P 29/00, A61P 25/04

(54) **1-[2H-1-BENZOPYRAN-2-ONE-8-YL]- PIPERAZINE DERIVATIVES FOR THE TREATMENT OF PAIN**
1-[2H-1-BENZOPYRAN-2-ON-8-YL]- PIPERAZIN- DERIVATE ZUR BEHANDLUNG VON SCHMERZEN
DÉRIVÉS DE LA 1-[2H-1-BENZOPYRAN-2-ONE-8-YL]-PIPERAZINE POUR LE TRAITEMENT DE LA DOULEUR

(30) Priority: 25.03.2004 US 555966 P; 25.03.2004 EP 04101230
(43) Date of publication of application: 20.12.2006
(73) Proprietor: Abbott Healthcare Products B.V., 1381 CP Weesp (NL)
(72) Inventor: BAKKER, Cornelis, IPSI Department, 1381 CP Weesp (NL); GLENNON, Jeffrey, 1381 CP Weesp (NL); HESSELINK, Mayke B., 1381 CP Weesp (NL); THAETE, Claudia, 1381 CP Weesp (NL); MCCREARY, Andrew, 1381 CP Weesp (NL); VAN SCHARRENBURG, Gustaaf J.M., 1381 CP Weesp (NL)
(74) Representative: Verhage, Marinus
(86) International application number: PCT/EP2005/051328
(87) International publication number: WO 2005/092340

(56) References cited:
- EP-A- 0 189 612
- EP-A- 0 372 657
- EP-A- 0 490 772
- EP-A- 0 650 964
- WO-A-00/39128
- WO-A-97/36893
- WO-A-99/52907

## Description

The invention relates to a novel use of known 1 -[2H-1-benzopyran-2-one-8-yl]-piperazine derivatives, broad spectrum 5-HT receptor binding compounds, having amongst other functional serotonin receptor activities, potent 5 -HT_{1A}-agonistic as well as 5-HT_{1D}-antagonistic activity. The invention also relates to the use of a compound disclosed herein for the manufacture of a medicament as specified in the claims. A beneficial effect is disclosed herein or apparent to a person skilled in the art from the specification and general knowledge in the art. In embodiments of the invention specific compounds disclosed herein are used for the manufacture of medicaments for pain.

Acute pain is a normal sensation triggered in the nervous system to alert an individual to possible injury. Chronic pain results from persistent pain signals in the nervous system which continue after the initial damage or injury has disappeared. Chronic pain can occur in the absence of any past injury or evidence of body damage, so-called psychogenic pain.

Many different types of analgesics exist. None of them is perfect, neither is it possible with the presently available drugs to adequately treat any type of pain, fast, and without any side-effects.

The invention had the object of providing a medicament for the treatment of pain containing at least one compound with a molecular mechanism of action different from that of all currently marketed analgesics, and thus of therapeutic value in pain conditions not satisfactorily treatable with known analgesics.

Surprisingly, the mono hydrochloric acid mono hydrate of 3-amino-8-(1-piperazinyl)-2H-1-benzopyran-2-one (hereafter named 'compound 1'), a broad spectrum 5-HT receptor binding compound, having amongst other functional serotonin receptor activities, potent 5-HT_{1A}-agonistic, 5-HT_{1D}-antagonistic activity, as well as 5-HT₇-agonistic activity (*see receptor binding profile, below*)*,* was found to be potently active in experimental animal models of pain. The compound is devoid of sedative effects when given in dosages of up to 100 mg/kg *p.o*., and was also shown to be highly active as inducers of growth factors. The latter activity is indicative of neuroprotective effects and improvement of brain plasticity required for neuroregeneration, and also indicative of potential therapeutic effects in neuropathic pain (see P. Anand., "Neurotrophic factors and their receptors in human sensory neuropathies", Prog. Brain Res., 146, 477-492, 2004; and R. Wang et al., "Glial cell line-derived neurotrophic factor normalizes neurochemical changes in injured dor sal root ganglion neurons and prevents the expression of experimental neuropathic pain", Neuroscience, 121, 815-824, 2003) and diabetes induced pain (J.A. Christianson et al., "Beneficial effects of neurotrophin treatment on diabetes-induced hypoalgesia in mice", J. Pain, 4, 493-504, 2003). When given orally, the compounds of the invention show a good bioavailability, which results in high potency and long duration of action.

The pharmacological activities as realized in the compounds of the invention an d their salts represents a novel class of analgesic compounds for the treatment of chronic pain disorders or in treating other conditions where there is hyper-sensitization to painful signals, hyperalgesia, allodynia, enhanced pain perception, and enhanced memory of pain.

The invention relates to compounds of the general formula (1) 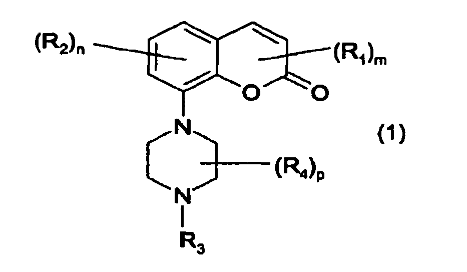 wherein:
- R₁: is alkyl(1-4C), alkoxy(1-4C), hydroxyl, alkoxy(1-4C)alkyl(1-4C), pyrrolidinyl, piperidinyl, morpholinyl, halogen, cyano, trifluoromethyl, amino, or mono-or disubstituted amino wherein the substituents are alkyl(1 -4C), or alkyl(1-4C) carbonyl,
- m: has the value 0, 1 or 2,
- R₂: is alkyl(1-4C), alkoxy(1-4C), halogen or trifluoromethyl,
- n: is 0 or 1, on the understanding that (m + n) is at least 1,
- R₃: is hydrogen, alkyl(1-3C) or alkenyl(2-3C)
- R₄: is alkyl(1-4C), and
- p: has the value 0, 1 or 2,
as well as pharmacologically acceptable salts thereof for use in the treatment of pain.

1-[2H-1-Benzopyran-2-one-8-yl]-piperazine derivatives, broad spectrum 5-HT receptor binding compounds, having amongst other functional serotonin receptor activities, potent 5-HT_{1A}-agonistic as well as 5 -HT_{1D}-antagonistic activity, were originally developed as antidepressants (EP 0 650 964). The presence of 5-HT_{1D} antagonism is thought to be of therapeutic value. 5-HT_{1D} recep tors are located presynaptically on the nerve terminal and have a negative modulatory influence on the release of 5-HT. Therefore, blockade of these receptors enhances the release of 5-HT from its terminals. The additional presence of presynaptic 5-HT_{1D} antagonism will result in a similar effect as observed after administration of 5-HT reuptake inhibitors. When 5-HT_{1D} antagonism is combined with 5-HT_{1A} agonism the later activity is strengthened.

That the above reasoning is likely to be valid for the analgesic activity of the compounds of the invention was shown by an interaction study with sumatriptan, the prototypical 5-HT_{1D} agonist developed as anti-migraine drug. Despite the fact that migraine often manifests itself as an excruciating pain in the head, 'pain' and 'migraine' most certainly are not synonymous. Until the development of the 'triptans' as selective anti-migraine drugs, the disorders was completely refractory to classical analgesics. The reverse is also applicable: 'triptans' are not analgesic. Sumatriptan was found to be completely Inactive in the 'hot plate' test (*see below*). Surprisingly however, it was found that surnatriptan nearly completely blocked the analgesic effect of compound 1 (*see below*), clearly demonstrating that the 5-HT_{1D}antagonism of this compound plays an important role in its analgesic activity.

WO 99/39128 and WO 99/52907 disclosed substituted azabicyclic compounds as selective agonists and antagonists of 5-HT₁ receptors, specifically one or both of the 5-HT_{1A} and 5-HT_{1D} receptors. Those compounds were claimed to be useful in treating or preventing migraine, depression and other disorders for which a 5-HT₁ agonist or antagonist is indicated.

To the invention belong all compounds having formula (1), racemates, mixtures of diastereomers and the individual stereoisomers. Thus compounds in which the substituents on potentially asymmetrical carbon atoms are in either the R-configuration or the S-configuration belong to the invention.

Pharmaceutically acceptable salts may be obtained using standard procedures well known in the art, for example by mixing a compound of the present invention with a suitable acid, for instance an inorganic acid such as hydrochloric acid, or with an organic acid. The active compounds and their salts can be processed to compositions by means of standard methods, for example pills, tablets, coated tablets, capsules, powders, injection liquids and the like, using auxiliary substances such as liquid and solid carrier materials.

Especially preferred are the compounds having formula (1) wherein (R₂)ₙ and (R₄)ₚ are hydrogen, R₃ has the meanings as given above, and (R₁)ₘ is a substituent at position 3 selected from the group consisting of pyrrolidinyl, piperidinyl, morpholinyl, amino and mono- or disubstituted amino wherein the substituents are alkyl(1 -4C) or alkyl(1-4C)carbonyl.

The invention particularly relates to the compound 3-amino-8-(1-piperazinyl)-2H-1-benzopyran-2-one and the salts thereof, i.e. the compou nd of formula (1) wherein (R₁)ₘ is 3-NH₂, (R₂)ₙ, R₃ and (R₄)ₚ are hydrogen, thus having formula (2):

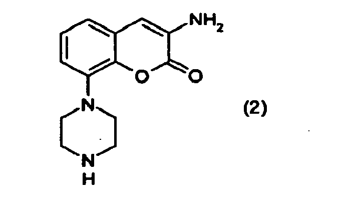

Especially preferred is the mono hydrochloric acid mono hydrate of 3-amino-8-(1-piperazinyl)-2H-1-benzopyran-2-one, hereafter referred to as 'Compound 1'.

The compounds of the invention are active at doses in the range of 0.1 - 100 mg/kg after oral administration, and their unique pharmacological profile makes them particularly useful in the treatment of pain.

As used herein the term pain shall refer to all types of pain. Preferably, the term shall refer to all types of chronic pain including nociceptive, neuropathic, psychogenic pain, and mixed category pain (nociceptive and neuropathic components). This in particular includes, but is not limited to, diabetic neuropathy, neurogenic pain, central pain, somatic pain, visceral and cancer pain, Inflammatory pain, post-operative pain, chronic low back pain, sciatica, cervical and lumbar pain, tension headaches, cluster headaches, chronic dally headaches, herpes neuralgia and post-herpetic neuralgia, facial and oral neuralgias and myofascial pain syndromes, phantom limb pain, stump pain and paraplegic pain, dental pain, opioid resistant pain, post-surgical pain including cardic surgery and mastectomy, pain of labour and delivery, post-partum pain, post-stroke pain, angina pain, genitourinary tract pain including pelvic pain and cystitis and vulvar vestibulitis and orchialgia, Irritable bowel syndrome, pre-menstrual syndrome pain, pain resulting from bums or chemical injury or sunburn, and bone injury pain.

Sub-types of nociceptive pain are somatic pain and visceral pain.

Somatic pain includes inflammatory pain, post-operative pain, chronic low back pain, cervical and lumber pain, cluster headaches, dental pain, pain of labour and delivery, postpartum pain, pain resulting from bums or chemical injury or sunburn, and bone injury pain.

Visceral pain includes cancer pain, post-surgical pain including cardic surgery, angina pain, genito-urinary tract pain including pelvic pain and cystitis and vulvar vestibulitis and orchialgia and pre-menstrual pain syndrome.

Sub-types of neuropathic pain are diabetic neuropathy, cancer pain, neurogenic pain, central pain, sciatica, herpes neuralgia, post-herpetic neuralgia, facial and oral neuralgias, phantom limb pain, stump pain and paraplegic pain, opioid -resistant pain, post-surgical pain including mastectomy and post-stroke pain.

Sub-types of psychogenic pain are chronic daily headaches and tension headaches.

Sub-types of mixed category pain are cancer pain, myofascial syndromes and tension headaches (*e.g.* McCaffery M, Pasero C. Pain:Clinical Manual p19 St. Louis: Mosby 1999*;* Merskek H and Bogduk (eds) Classification of chronic pain, 2nd edition, IASP Task Force on Taxonomy, p 209-214, IASP Press, Seattle 1994*;* The Merck Manual, Section 14, Chapter 167, Pain, 17Edition Merck & Co 1999).

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars or sugar alcohols, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the Ingredients of a pharmaceutical composition of the invention. Associated with such container(s) can be various written materials such as instructions for use, or a notice In the form prescribed by a governmental agency regulating the manufacture, use or sale of pharmaceuticals products, which notice reflects approval by the agency of manufacture, use, or sa le for human or veterinary administration.

### PROTOCOLS FOR THE PHARMACOLOGICAL ASSAYS

### CHRONIC CONSTRICTIVE NERVE INJURY: A NEUROPATHIC PAIN MODEL

The objective of these studies is to evaluate the potential analgesic properties of a test substance in the Bennett and Xie model of peripheral mononeuropathy (see: G.J. Bennett and Y-K. Xie, "A peripheral mononeuropathy in rat that produces disorders of pain sensation like those seen in man", Pain, 33, 87-107, 1988).

### Study Design

Male Sprague-Dawley rats (approximately 250g; Harlan, UK, or other accredited supplier). The observer is blind to the treatment groups.

Five treatment groups (n=10 rats/group); vehicle control, reference substance and 3 dosing groups for test substance. 2 days baseline testing, surg ery to induce a peripheral mononeuropathy and monitoring of the development of the neuropathy using behavioural testing on days 10 and 11 post-operatively (PO). Chronic drug administration, subcutaneous (either by twice daily injection, or continuous infu sion via osmotic mini-pumps), starting on day 12 PO for 14 days and behavioural testing at four time points post-dose for mechanical allodynia.

### Procedure:

Rats are prepared in batches (with members of each treatment group in each batch), and the behavioural tests and dosing is run at fixed intervals after surgery for each batch. The behavioural tests (see below) are performed on all rats for a period of 2 days prior to surgery, to establish base-line values. A peripheral mononeuropathy is then induced by placing four loosely constrictive ligatures around the right common sciatic nerve under aseptic conditions. The animals are allowed to recover from surgery for a minimum of 4 days before the behavioural testing is recommenced (B. Lynn and S.E. Carpenter, "Primary afferent units from the hairy skin of the rat hind limb", Brain Research, 238, 29-43, 1982)

The behavioural testing is resumed on day 10 post-operatively (PO), and is repeated on day 11 to monitor the development of allodynia. Drug treatment is carried out from day 12 PO (the time-point corresponding to maximal behavioural changes), or as defined in the study protocol, and a time -course of behavioural tests is carried out. Animals showing any signs of autotomy of the affected digits are terminated. Any animal which does not develop a peripheral mononeuropathy (as determined by the results of the behavioural tests employed in a particular protocol) is not used in the study.

### Behavioural Tests:

Mechanical allodynia test: the animal is placed in a wire mesh cage and a series of Von Frey filaments are applied to the plantar surface of the hind paw, from below. The filaments are applied in ascending order (starting with the weakest force), and the withdrawal threshold for both the ipsilateral and contralateral hind paws are evaluated. The withdrawal threshold is defined as being the lowest force of two or more consecutive Von Frey filaments to elicit a reflex withdrawal response (i.e. a brief paw flick).

### Analysis of Results:

Standard statistical methods are employed to evaluate test substance related effects. Data are analysed for homogeneity and either parametric or non -parametric methods applied as appropriate.

### LIGATURE OF SPINAL NERVES: A NEUROPATHIC PAIN MODEL

Neuropathic pain test (Chung test) in the rat according to Kim and Chung (Pain 1992, 50: 355-363): tight ligature of spinal nerves in rats is associated with hyperalgesia, allodynia and spontaneous pain, and therefore constitutes a model for peripheral neuropathic pain in humans. Antihyperalgesics reduce these chronic signs of pain hypersensitivity. Rats (180-220 g) are anesthetized (sodium pentobarbital 40 mg/kg *i.p.*) and an incision at the L4-S2 level is performed to expose the left L5 and L6 spinal nerves. A ligature is tied tightly around each nerve. The wound is then sutured. The rats receive an intra muscular *(i.m.)* injection of 50 000 IU Penicilline and are allowed to recover. At least 2 weeks after the surgery, when the chronic state is fully installed, rats are submitted consecutively to thermal and tactile stimulation of both the non-lesioned and the lesioned hindpaws. For thermal stimulation, the apparatus consists of 6 Individual Plexiglas boxes (17 × 11 × 13 cm) placed upon an elevated glass floor. A rat is placed in the box an d left free to habituate for 10 minutes. Then, a mobile infrared radiant source (setting 20) is focused under the non-lesioned and lesioned hindpaws and the paw-withdrawal latencies are automatically recorded. Paw-withdrawal interrupts the reflected radiation and switches off the counter and the light source. In order to prevent tissue damage, if no reaction is noted, the test is terminated after 45 seconds. For tactile stimulation, the animal Is placed under an inverted Plexiglas box (17 × 11 × 13 cm) on a grid floor. The tip of an electronic Von Frey probe is then applied with increasing pressure to the non-lesioned and lesioned hindpaws and the force required to induce paw-withdrawal is automatically recorded. This procedure Is carried out 3 times and the mean force per paw is calculated to provide basic scores per animal. Prior to receiving drug treatment all animals will be submitted to tactile stimulation and assigned to treatment groups matched on the basis of their pain response.

### IN VIVO ELECTROPHYSOLOGY: A MODEL FOR INFLAMMATORY PAIN

In vivo electrophysiology is a powerful means of looking directly at the sensory responses of spinal neurones to suprathreshold stimuli (unlike behavioral tests that use thresholds) and so are more related to clinical pain states.

Male Sprague-Dawley rats (200-250g) are anaesthetised with 2-3% halothane (in 66% N₂O and 33% O₂) and subsequently maintained at 1.8% halothane. Core temperature of the animal is monitored using a rectal thermometer probe coupled to a heating blanket. At the end of the experiment, animals are killed with an overdose of anaesthetic. A laminectomy is performed to expose the segments L4-L5 of the spinal cord, and a parylene-coated tungsten electrode is descended into the dorsal hom using an Epson Stepper device. The depth of the recording site is noted from the microdrive readings. Extracellular recordings are made from single dorsal hom neurones receiving C- and A-fibre input from the skin of the hindpaw, identified by their ability to respond to both noxious and innocuous stimuli (pinch and touch). Neuronal responses are elicited by transcutaneous electrical stimulation given in the centre of the receptive field of the neurone in the ipsilateral hindpaw, at 3 times the threshold current required for C-fibre evoked activity. At 10-minute intervals, tests consisting of a train of 16 stimuli (2msec-wide pulses at 0.5Hz) are carried out and post-stimulus histograms constructed. These evoked responses are separated, according to latency, into Aβ-fibre evoked activity (0-20 msec post-stimulus); Aδ-fibre evoked activity (20-90 msec); C-fibre evoked activity (90-300 msec) and post-discharge of the neurone (300-800 msec). The neuronal response evoked by the first stimulus of the train is referred to as "input" and consists of the number of action potentials (90-800msec) evoked by this stimulus. Wind-up, a measure of the enhanced neuronal response elicited by repetitive stimulation, is quantified as the difference between the total number of action potentials produced by the 16 stimuli (90-800 ms), and the input × 16. Thus the measure of wind-up includes both the enhanced C-fibre evoked responses and the post-discharge generated as wind-up develops. Von Frey hairs (1-75g) and heat (30-48°C) are also used to quantify responses to natural mechanical and thermal stimuli.

Tests are performed at 10-minute intervals, until the neuronal responses evoked at each test differed by less than 10%. The results of the last three tests are then averaged to give control values for each parameter.

Cumulative doses of the compound (in 50 µl volume) are then applied to the exposed spinal cord into a 'well' formed by the laminectomy which held the 50 µl volume of drug. The neuronal responses are followed for 60 minutes after each dose, after which the solution can be removed and the next dose applied. Similarily, systemic effects can be measured using subcutaneous injection in 0.2ml.

Data are shown as percentage of pre-drug control values, with a 60 minute time-course for each dose of the drug. To generate a dose response curve, the maximum effects of each dose on each neurone are averaged. Data are presented as mean ± s.e.m. 10 Neurones are needed for each route. A comparison is made with the effects of the compound (by the most effective route as determined by the above studies) in normal animals with that seen 3 hours after carrageenan inflammation. Thus a total of 30 experiments are done - one neurone per animal and each study lasts one day.

### FORMALIN PAW TEST: A MODEL FOR INFLAMMATORY PAIN

Formalin paw test in the mouse or rat accorrding to Wheeler-Aceto et al., (Psychopharmacology 1991, 104: 35-44)*:* animals are given an Intraplantar injection of 5% formalin (25 µl for the mouse, 50 µl for the rat) into the posterior left paw. This treatment induces a recognizable flinching response in control animals. The number of flinches is counted for 10 minutes, beginning immediately after injection of formalin (early phase) and again for 5 minutes in mice or 15 minutes in rats, beginning 20 minutes after the injection.

### CARRAGEENAN EDAMA TEST: A MODEL FOR INFLAMMATORY PAIN

Carrageenan Edema Test in the rat follows that described by Winter et al. (Proc.Soc. Exp. Biol. Med. 1962, 111: 544-547)*:* solution into the lower surface of the right hind - paw (0.75 mg per paw in 0.05 ml physiological saline). 2 hours later rats are submitted consecutively to thermal and tactile stimulation of both the non -inflamed and the inflamed hindpaws. For thermal stimulation, the apparatus (Ugo Basile, Reference: 7371) consists of 6 individual Plexiglas boxes (17 x 11 x 13 cm) placed upon an elevated glass floor. A rat is placed in the box and left free to habituate for 10 minutes. Then, a mobile infrared radiant source (setting 20) is focused under the non-inflamed and inflamed hindpaws and the paw-withdrawal latencies are automatically recorded. Paw-withdrawal interrupts the reflected radiation and switches off the counter and the light source. In order to prevent tissue damage, if no reaction is noted, the test is terminated after 45 seconds. For tactile stimulation, the animal is placed under an inverted Plexiglas box (17 × 11 × 13 cm) on a grid floor. The tip of an electronic Von Frey probe is then applied with increasing pressure to the non - inflamed and inflamed hindpaws and the force required to induce paw-withdrawal is automatically recorded. This procedure is carried out 3 times and the mean force per paw is calculated to provide basic scores per animal. 3.5 hours later, the animals are sacrificed by a blow to the cervical vertebrae and the hind-paws sectioned and weighed. An increase in paw weight (edema) indicates inflammation. This later procedure can also be appiled to mice.

### YEAST HYPERTHERMIA TEST: A MODEL FOR INFLAMMATORY PAIN

Yeast Hyperthermia Test in the mouse or rat according to by Teotino et al (J. Med.Chem. 1963, 6: 248)*:* Animals are first measured for rectal temperature using a rectal probe. They are then injected with a yeast suspension (512 mg/kg *s.c*.). 8 hours later, the test substance is administered. Mice are measured for rectal temperature immediately before test substance administration and again 60 and 120 minutes later.

### COLERECTAL DISTENSION IN RATS: A MODEL FOR VISCERAL PAIN

### Experimental Procedure:

Animals (Female Sprague Dawley rats, body weight: 208 - 257 g, five to seven per group) are fasted for 24 hours prior to the experiments with free access to water. Acetic acid (0.6 %. 1.5 ml) is injected into the colon (10 cm proximal to the anus). After 50 minutes a rubber balloon of 5 cm length (6-7ml volume) is inserted rectally into the descending colon and secured by taping the attached tubing to the rat's tail. The balloon pressure Is set to 100 mbar for 10 minutes. During this time the number of abdominal constrictions is recorded by visual inspection. The experiments are continued only in animals which respond to the colorectal distention with more than 10 abdominal constrictions. These animals receive a single subcutaneous dose of an active compound or vehicle (10% Arlatone G, 10% Ethanol (96 %) in Water) and the colorectal distention protocol is repeated at 30, 60, 90 and 120 min after administration.

### Data analysis:

Results are given as mean ± SD. The number of abdominal constrictions at 30, 60, 90 and 120 min after administration of substance or vehicle as well as the mean values (30-120 min) are compared to prevalues (time 0) by paired two sided t-tests. Values of p<0.5 are taken as statistically significant.

Furthermore, the relative number of constrictions (% of prevalues) Is calculated for each animal and the mean values of the groups are given.

### NEUROPROTECTIVE ACTIVITY: INDUCTION OF GROWTH FACTORS

Compounds of the invention (3 mg/kg, *p.o*.) or vehicle are administered once daily during a period of 3 weeks (n=8 animals per treatment group). 24 hours after the last dose the animals are sacrificed (using CO₂/O₂ anesthesia), the brains are removed and the dissected. RNA is extracted from the individual brain samples and induction of the growth factors BDNF and GDNF is determined by quantitative PCR. Total RNA is isolated with the Trizol method (Invitrogen) from the brain pieces. cDNA is made starting with 2 µg of total RNA (pretreated for 30 min with DNAse (Ambion) in first strand buffer) using the reverse transcriptase Superscript II (Invitrogen). Quantification of mDNA by real time PCR makes use of the observation that the early cycles of PCR are characterized by an exponential increase in target amplification. The accumulation of PCR product is measured using Sybergreen II. Primers are designed using the software package Primer Express (Applied Biosystems). Expression levels of the housekeeping genes ornithine decarboxylase (ODC_ex8) and alpha tubulin (TUBA) are used for normalization and as control for good cDNA synthesis.

### HOT PLATE TEST: A MODEL FOR ACUTE PAIN

Hot plate test in mouse or rat according to Eddy and Lelmbach (J. Pharmacol. Exp.Ther. 1953, 107: 385-393*):* Mice or rats are placed onto a hot metal plate maintained at 54°C for mice or 52°C. for rats surrounded by a Plexiglas cylinder (Height: 13 cm; Diameter: 19 cm). The latency to the first foot -lick is measured (maximum: 30 seconds).

### TAIL FLICK TEST: A MODEL FOR ACUTE PAIN

Tail flick test in mouse or rat according to by D'Amour and Smith (J. Pharmacol.Exp. Ther.1941, 72: 74-79*):* The animal's tail is heated by means of a thermal light source. The latency before the animal withdraws its tail is measured (maximum: 15 seconds for mice, 30 seconds for rats).

### PHENYLBUTAZON/ACETIC ACID WRITHING TESTS: MODELS FOR ACUTE PAIN

Phenylbenzoquinone and acetic writhing tests in mice follow the methods described by Hendershot et al (J. Pharmaco/. Exp. Ther. 1959, 125: 237-240*):* Mice are injected with phenylbenzoquinone (PBQ) (1.25 mg/kg *i.p*.) or acetic acid *(0.5% I.p*.). This treatment Induces a recognizable writhing response In control animals. The number of writhes is counted for 10 minutes beginning 5 minutes after injection of PBQ or acetic acid.

### FREUNDS'S ADJUVANT TEST: A MODEL FOR CHRONIC INFLAMMATORY PAIN

Chronic inflammatory pain test (Freund's adjuvant test) in the rat according to Whiteley (Current Protocols In Pharmacology, Wiley, N.Y., 5.5, 1999): an injection of Freund's adjuvant in rats induces chronic clinical signs of polyarthritis with pain. On Day 1, rats are weighed and injected intradermally with a suspension of Mycobacterium butyricum (Freund's adjuvant) into the proximal quarter of the tall (1 mg in 0.1 ml mineral oil). Sham controls receive a similar injection of mineral oil. On Day 18, when the chronic state is fully installed, rats are weighed again and are evaluated for clinical symptoms of inflammation. They are then submitted consecutively to thermal and tactile stimulation of both hindpaws. For the clinical signs, each paw is scored for inflammation according to a 5-point scale (0-4) and the tail according to a 4-point scale (0-3), i.e. a maximum score of 19 per animal. For thermal stimulation, the apparatus (Ugo Basile, Reference: 7371) consists of 6 individual Plexiglas boxes (17 × 11 × 13 cm) placed upon an elevated glass floor. A rat is placed in the box and left free to habituate for 10 minutes. Then, a mobile infrared radiant source (setting 20) is focused under each hindpaw and the paw-withdrawal latency is automatically recorded. Paw-withdrawal interrupts the reflected radiation and switches off the counter and the light source. In order to prevent tissue damage, if no reaction is noted, the test is terminated after 45 seconds. For tactile stimulation, the animal is placed under an inverted Plexiglas box (17 × 11 × 13 cm) on a grid floor. The tip of an electronic Von Frey probe (Bioseb, Model 1610) is then applied with increasing pressure to each hindpaw and the force required to induce paw-withdrawal is automatically recorded. This procedure is carried out 3 times and the mean force per paw is calculated to provide basic scores per animal. Prior to receiving drug treatment all animals will be submitted to tactile stimulation and assigned to treatment groups matched on the basis of their pain response.

### RECEPTOR BINDING EXPERIMENTS

Receptor binding data were obtained by CEREP (128, rue Danton, 92500 Rueil-Malmalson, France) or at Solvay Pharmaceuticals B.V., using well documented standard procedures. Affinity for 5-HT1A receptors for instance, was determined by testing the ability of the compounds of the invention to displace [³H]-2-(di-n-propylamino)-8-hydroxytetralin ([³H]-8-OH-DPAT) from Its specific binding sites in rat frontal cortex homogenates. This test Is based on the method described by Gozlan et al. (Nature, 305, (1983), pages 140-142).

### DOSE

The affinity of the compounds of the invention for serotonin receptors was determined as described above. From the binding affinity measured for a given compound of formula (1), one can estimate a theoretical lowest effective dose. At a concentration of the compound equal to twice the measured K₁-value, 100% of the receptors are likely to be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### EXAMPLE I. ANALYTICAL METHODS USED DURING SYNTHESES

### NUCLEAR MAGNETIC RESONANCE (NMR) SPECTROSCOPY

NMR spectra were recorded on a Bruker AM400 spectrometer, or a Varian VXR400S spectrometer. Chemical shifts (δ) were reported in ppm downfield from TMS as internal standard. A sample of 10-50 mg was dissolved in a deuterated solvent, usually CDCl₃ or a DMSO-d6/CDCl₃ (4:1 v/v) mixture). The solvent was selected to ensure complete dissolution of the sample. The free induction decays were generally obtained at room temperature under the following conditions:
- Digital resolution: : 0.2 Hz
- Sweep width: : 18 ppm
- Pulse width: : 20 degrees
- Pulse repetition time: : 4.5 sec or longer if required for complete relaxation
- Carrier frequency: : 6.0 ppm
- Number of acquisitions: : 128 or more if necessary. The C-13 satellite signals at 0.5% signal intensity should be dearly visible.

NMR was used as method for determining relative contents.

### TITRIMETRY (CHLORIDE AND WATER DETERMINATIONS)

For potentiometric titrations, a Metrohm model E636 (Switzerland) was used.

Potentiometric chloride determinations were used in this syntheses to determine chloride. The titration was performed with a combined silver electrode and silver nitrate titrant. The method is specific for chloride because it can distinguish chloride from iodide and bromine on basis of different electrode potentials.

Voltametric titrations for the determination according to Karl Fisher were performed using a Metrohm 633KF (Metrohm, Switzerland) apparatus according to the USP method.

### EXAMPLE II. SYNTHESIS OF 3-AMINO-8-(1-PIPERAZINYL)-2H-1-BENZOPYRAN-2-ONE AND ITS MONOHYDROCHLORIC ACID MONOHYDRATE (Compound 1)

### STEP 1: NITRATION

The first step was the nitration of 5-bromo-2-hydroxybenzaldehyde **(1*)** yielding 5-bromo-2-hydroxy-3-nitrobenzaldehyde **(2*)**:

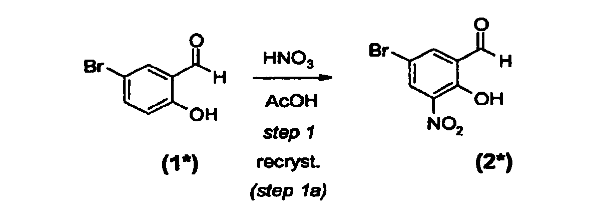

A solution of 1.0 mol of 5-bromo-2-hydroxybenzaldehyde **(1*)** in 3.75 litres acetic acid (98%) was formed on heating the mixture to about 60°C. 1.5 mol of concentrated nitric acid (137 g = 97 ml) was added slowly in approximately 1 hour. After the completion of the addition stirring was continued at 65°C for a further 10 minutes. The solution was then cooled to 45 °C, and the product was precipitated by the addition of 4 litres of water. After stirring for at least 3 hours the product was collected on a filter and washed with water until the pH of the mother liquor was approximately 6. The material is dried as much as possible by centrifugation. The crude product was dissolved in 800 ml acetone under refluxing and stirring. 400 ml acetone was removed by distillation. After cooling to 20°C, the mixture was stirred for 3 hours. The precipitate was collected on a filter an d washed with petroleum ether 40-65°C. The solid was dried overnight in an air stream at 40°C. Finally, the crude **(2*)** was recrystallized from acetone to yield an end product with a purity of 98% as shown by NMR analysis.

5-bromo-2-hydroxybenzaldehyde **(1*)** was identified by its characteristic chemical shift δ 9.84 ppm; 5-bromo-2-hydroxy-3-nitrobenzaldehyde **(2*)** had a characteristic chemical shift of δ 10.4 ppm.

The overall yield of this step was approximately 60% (crude on crude).

### STEP 2: ERLENMEYER CONDENSATION

The second step was the Erlenmeyer condensation of 5-bromo-2-hydroxy-3-nitrobenzaldehyde **(2*)** with N-acetyl-glycine to yield N-(6-bromo-8-nitro-2-oxo-2H-1-benzopyran-3-yl)acetamide **(3*)**.

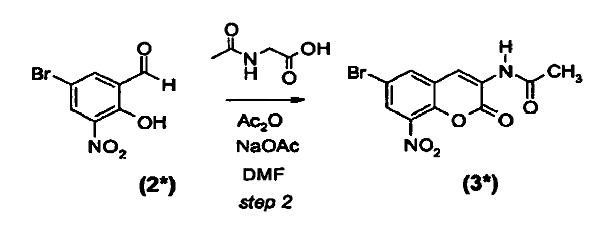

To a mixture of 1.0 mol of 5-bromo-2-hydroxy-3-nitrobenzaldehyde **(2*)**, 1.0 mol of N-acetylglycine and 1.0 mol of anhydrous sodium acetate, 800 ml of N -methyl-2-pyrrolidone are added. The mixture was stirred and heated to 50°C. Then 2.2 mol of acetic anhydride was run into the reaction vessel in approximately 30 minutes. The reaction mixture was heated to 100°C. During heating the reacting mixture became homogeneous for a while; shortly afterwards a solid was formed, making stirring troublesome. After heating at 100°C for 4 hours, the mixture was cooled to 80°C and 1,100 ml of acetic acid (98%) was added. Thereafter stirring of the mixture was easy. Next, the mixture was cooled to room temperature, and stirred for 60 minutes. The precipitate was collected on a filter and washed twice with 625 ml acetic acid (80%), five times with 900 ml water, and once with 300 ml acetone. The product was dried in an air stream at 40°C for 24 hours, and had a purity of 98% as shown by NMR analysis.

5-bromo-2-hydroxy-3-nitrobenzaldehyde **(2*)** had a characteristic shift of δ 10.4 ppm; the characteristic chemical shift of N-(6-bromo-8-nitro-2-oxo-2H-1-benzopyran-3-yl)acetamide **(3*)** was δ 8.72 ppm

The overall yield of this step was approximately 80% (crude on crude).

### STEP 3: REDUCTION

The third step was the catalytic hydrogenation of N-(6-bromo-8-nitro-2-oxo-2H-1-benzopyran-3-yl)acetamide **(3*)** to N-(8-amino-2-oxo-2H-1-benzopyran-3-yl)-acetamide **(4*)**.

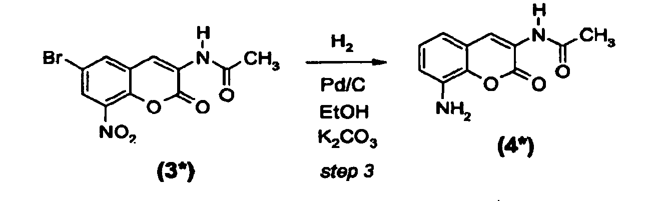

A mixture of 1.0 mol of N-(6-bromo-8-nitro-2-oxo-2H-1-benzopyran-3-yl)acetamide **(3*)**, 50 g of 10% palladium on carbon paste (containing 61% water), 1.0 mol of potassium carbonate and 15 litre of ethanol was heated to 60°C. At this temperature the starting material was reduced with hydrogen at an overpressure of 4 bar at 1400 rpm. After completion of the reaction (1 hour), the catalyst was removed by filtration using filterald, and washed with 4.5 litre methyl ethyl ketone (MEK). The filtrate was concentrated to 2 litre, and 2.3 litre of MEK was added In order to change the solvent from ethanol to MEK, 2 litre of the solvent mixture was distilled off at normal pressure and 2 litre of MEK was added. This was repeated 4 times. Then 5 litre of MEK and 2.6 litre of water were added and the mixture was stirred. The layers were separated. The upper later was concentrated at normal pressure to approximately 3.5 litre. The residue was cooled to 25°C. During this cooling the product crystallized. Then the mixture was cooled to -10°C and stirred for two hours. The solid was filtered and washed three times with 800 ml hexane. The product was dried (50°C, 20 cm Hg, N₂) until constant weight.

N-(6-bromo-8-nitro-2-oxo-2H-1-benzopyran-3-yl)acetamide **(3*)** had a characteristic chemical shift of δ 8.72 ppm: that of N-(8-amino-2-oxo-2H-1-benzo-pyran-3-yl)-acetamide **(4)** was δ 8.55 ppm

The overall yield of this step was approximately 70% (crude on crude).

### STEP 4: CONSTRUCTION OF PIPERAZINE RING SYSTEM

Step 4 was the alkylation of N-(8-amino-2-oxo-2H-1-benzopyran-3-yl)-acetamide **(4*)** with bis-chloroethylamine yielding N-(8-(1-piperazinyl)-2-oxo-2H-1-benzopyran-3-yl-) acetamide **(5*)**.

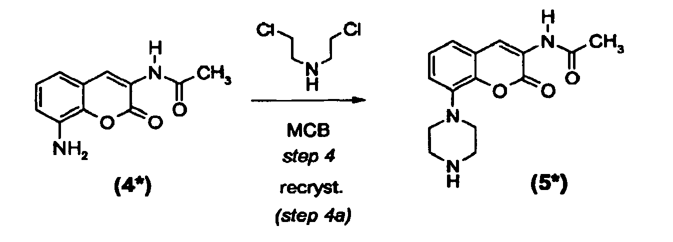

A mixture of 2.5 litre monochlorobenzene, 1.0 mol of N-(8-amino-2-oxo-2H-1-benzopyran-3-yl)-acetamide **(4*)** and 1.2 mol bischloroethylamine hydrochloride was heated to reflux under nitrogen. Part of the monochlorobenzene (0.5 litre) was distilled off. This mixture was refluxed for 10 days. The reaction was followed by HPLC. After the reaction, the mixture was cooled to 20°C and stirred overnight. The solid product was collected on a filter and washed once with 360 ml monochlorobenzene and 3 times with 360 ml ethanol. The product was dried in vacuum at 50°C.

Half of the crude product was dissolved in 3 litre water. After addition of 18 g of Celite and 50 g of charcoal, the mixture was stirred for 1 hour at room temperature. After filtration the solution was concentrated by distillation of water. In the mean time the second half of the crude product was treated as described above. When the total volume of the combined aqueous solutions was about 1.5 litre, distillation was stopped and the mixture was cooled to room temperature. Then 125 g sodium bicarbonate was added in portions. After stirring for 1.5 hours at 15°C the precipitate formed was collected on a filter. After washing with 360 ml water and 2 times with 270 ml ethanol, the product was dried in vacuum at 50 °C. N-(8-amino-2-oxo-2H-1-benzo-pyran-3-yl)-acetamide **(4*)** had a characteristic chemical shift of δ 8.55 ppm; that of N-(8-(1-piperazinyl)-2-oxo-2H-1-benzopyran-3-yl-)acetamide **(5*)** was δ 8.57 ppm.

The overall yield of this step was approximately 50% (crude on crude).

### STEP 5: AMIDE HYDROLYSIS

Step 5 was the hydrolysis of the amide function of N-(8-(1-piperazinyl)-2-oxo-2H-1-benzopyran-3-yl-)acetamide **(5*)** using hydrochloric acid. This resulted In the trihydrochloric acid salt of 3-amino-8-(1-piperazinyl)-2H-1-benzopyran-2-one **(6*)**.

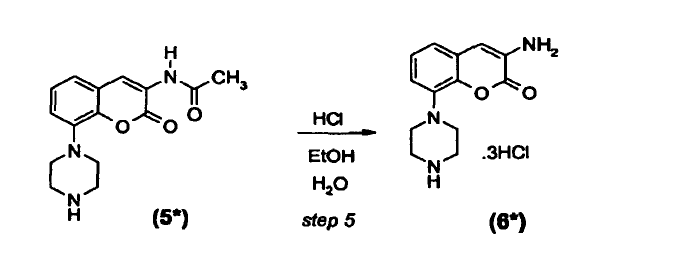

2.9 Litre of concentrated hydrochloric acid was added at room temperature to a suspension of 1.0 mol of N-(8-(1-piperazinyl)-2-oxo-2H-1-benzopyran-3-yl-) acetamide **(5*)** and 1.4 litre of absolute ethanol in about 10 minutes. During this addition the temperature rose to 40°C. After the addition the mixture was stirred at a temperature of 50°C during 1.5 hours. The mixture was cooled to 20°C and, after crystallisation had started, 1.4 litre of absolute ethanol was added. Then the mixture was stirred for 1 hour at 20°C and for 2 hours at 0°C. The crystals were isolated by filtration and washed twice with 0.6 litre of acetone. The isolated product was dried in vacuum (40°C, 200 mm Hg, N₂, 24 hours).

N-(8-(1-piperazinyl)-2-oxo-2H-1-benzopyran-3-yl-)acetamide **(5*)** had a characteristic chemical shift of δ 8.57 ppm; the trihydrochloric acid salt of 3-amino-8-(1-piperazinyl)-2H-1-benzopyran-2-one **(6*)** had a characterristic chemical shift of δ 6.77 ppm.

The overall yield of this step was approximately 85% (crude on crude).

### STEP 6: PARTIAL NEUTRALISATION

The final step, the sixth, was the partial neutralisation of the trihydrochloric acid salt **(6*)** with sodium bicarbonate to produce the desired product: COMPOUND 1, the mono hydrochloric acid mono hydrate of 3-amino-8-(1-piperazinyl)-2H-1-benzopyran-2-one **(7*)**

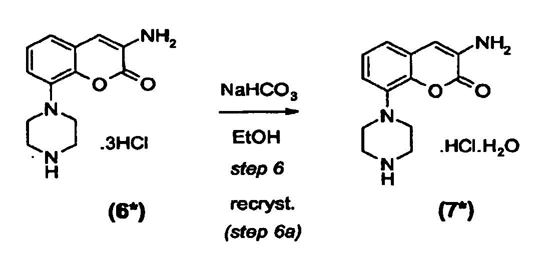

To a suspension of 1.0 mol of the trihydrochloric acid salt **(6*)** in 3.5 litre ethanol a solution of 2.2 mol sodium bicarbonate in 2.8 litre water was added in about 30 minutes. The temperature was between 20°C and 25°C. The suspension was then stirred for 3 hours. The reaction mixture was filtered and subsequently washed with 1.1 litre water, 1.1 litre ethanol and 1.1 litre hexane. The isolated crude product was dried in vacuum (40°C, 200 mm Hg, N₂, 24 hours).

The dried product (1 mol) was dissolved in 9 litre methanol by heating to reflux temperature. The solution did not become completely clear. After cooling to 20°C the mixture was filtered. 300 ml of water and 150 ml of methanol was added to the filtrate, after which about 3 litre of the solvent mixture was distilled at normal pressure. The complete procedure was repeated with another mol of the dried product. Then the combined fractions wre concentrated to a volume of about 12 litre by distillation. After addition of 6 litre ethanol, 6 litre of the solvent mixture was removed by distillation at normal pressure. The mixture was then cooled to 0°C and stirred for 2 hours. The precipitate was collected on a filter and washed twice with 750 ml acetone. The product was dried under vacuum (40°C, 200 mm Hg, N₂, 24 hours), and thereafter homogenized by milling and, when necessary, by micronizing.

The trihydrochloric acid salt of 3-amino-8-(1-piperazinyl)-2H-1-benzopyran-2-one **(6*)** had a characteristic chemical shift of δ 6.77 ppm; that of the endproduct, COMPOUND 1, was δ 6.7 ppm.

The overall yield of this step was approximately 85% (crude on crude).

COMPOUND 1, the mono hydrochloric acid mono hydrate of 3-amino-8-(1-piperazinyl)-2H-1-benzopyran-2-one, had a molecular formula C₁₃H₁₈ClN₃O₃ and a molecular mass of 299.5. The pure product (99.8%, NMR) was a white to yellowish powder. Its chloride content was 11.7% (mass to mass), as determined by titrimetry. Its water content, determined by Karl Fisher water assay titration, was 6.5% (mass to mass).

### EXAMPLE III: FORMULATION OF COMPOUND 1

For oral *(p.o.)* administration: to the desired quantity (0.5-15 mg) of Compound 1 in a glass tube, some glass beads were added and the substance was milled by vortexing for 2 minutes. After addition of 1 ml of a solution of 1% methylcellulose in water, the compound was suspended by vortexing for 10 minutes. For concen - trations up and above 1 mg/ml remaining particles In the suspension were further suspended by using an ultrasonic bath.

**For Intraperitoneal *(i.p.)* administration:** to the desired quantity (0.5-15 mg) of the solid compound 1 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE IV: PHARMACOLOGICAL TESTRESULTS

### COLERECTAL DISTENSION IN RATS: A MODEL FOR VISCERAL PAIN

Colorectal distention after sensitization with acetic acid (*according to the protocol given above*) resulted in comparable numbers of abdominal constrictions in all groups. The lowest dose of Compound 1 (0.1 µmol/kg) was dissolved in 10% Arlatone G, 10% Ethanol (96%) in Water and reduced the visceral hypersensitivity at 90 min after administration. In a control group a comparable vehicle was tested (20% Arlatone G, 20% Ethanol (96%) in water). No significant reduction of the number of abdominal constrictions during the repetitive colorectal distention protocol was observed. Compound 1 exhibited a significant inhibition of the number of abdominal contractions due to colorectal distention after sensitization with acetic acid in the dose range between 3 and 10 µmol/kg 90 minutes after subcutaneous administration. The vehicle used for this dose did not interfere with the experimental protocol.

### INDUCTION OF GROWTH FACTORS

Treatment with Compound 1 increased GDNF and BDNF RNA levels in the thalamus, striatum, prefrontal cortex, nucleus accumbens and hippocampus (see table)

Regulation of growth factor RNA by treatment with Compound 1 as determined by quantitative PCR. Data are expressed as fold increase compared to vehicle treatment.

| Brain area | GDNF | BDNF |
|---|---|---|
| thalamus | **2.6 (p<0.001)** | **2.3 (p0.0001)** |
| striatum | **2.2 (p<0.00005)** | 1.8 (p=0.12) |
| prefrontal cortex | 1.3 (p=0.24) | **2.1 (p<0.0005)** |
| nucleus accumbens | 1.5 (p<0.05) | 5.1 (p<0.05) |
| hippocampus | **1.7 (p<0.001)** | **2.4 (p<0.0001)** |

### HOT PLATE TEST: A MODEL FOR ACUTE PAIN

Compound 1 and other compounds were tested on the hotplate 30 minutes after intraperitoneal administration. A description of the method is given above.

| **Drug treatment** | **Latency of animal to remove itself from the hotplate seconds + STDerr** |
|---|---|
| **saline** | **4.5 ± 0.3** |
| | |
| **morphine (7.5. mg/kg i.p.)** | **7.8 ± 0.3** |
| | |
| **compound 1 (0.1 mg/kg i.p.)** | **8.4 ± 0.5** |
| | |
| **sumatriptan (10 mg/kg i.p.)** | **3.9 ± 0.3** |
| | |
| **comp 1 (0.1 i.p.) + sumatriptan (10 i.p.)** | **4.7 ± 0.3** |

From the data given in the table above it is clear that morphine nearly doubles the latency of the animals to remove themselve from the hotplate: a pertinent analgesic effect. Compound 1 is as effective as morphine, but at a much lower dose. The selective 5-HT_{1D} agonist sumatriptan is inactive at the very high dose of 10 mg/kg i.p., but at that dose it nearly completely antagonizes the analgesic effect of compound 1 (P-value = 0.0001, Wilcoxon test).

Compound 1 is also orally active in this test in mice: its' ED₅₀ is 3.2 mg/kg (*p.o*.)

### ACETIC ACID WRITHING TEST: MODEL FOR ACUTE PAIN

Compound 1 is active in this test: its' ED₅₀ is 1.82 mg/kg *p.o*.

### RECEPTOR BINDING PROFILE OF COMPOUND 1.

The binding data collected in the table below were either obtained by CEREP (128, rue Danton, 92500 Rueil-Malmaison, France) or at Solvay Pharmaceuticals B.V., using well documented standard procedures.

| **receptor** | **S¹** | **radioligand** | **Kₗ(nM) Compound 1** |
|---|---|---|---|
| | | | |
| 5-HT_{1A} | h | [¹H]-8-OH-DPAT | **0.25** |
| 5-HT_{1B} | r | [¹²⁵I]-cyanopindolol | **2.0** |
| 5-HT_{1D} | b | [³H]-serotonin | **13** |
| 5-HT_{2A} | h | [³H]-ketanserin | **630** |
| 5-HT_{2B} | h | [³H]-LSD | **320** |
| 5-HT_{2C} | h | [¹²⁵I]-DOI | **> 1,000** |
| 5-HT₃ | h | [³H]-BRL 43694 | **250** |
| 5-HT₄ | h | [³H]-GR 113808 | **> 1,000** |
| 5-HT₆ | h | [³H]-LSD | **100** |
| 5-HT₆ | h | [³H]-LSD | **> 1,000** |
| 5-HT₇ | h | [³H]-LSD | **3.2** |
| 5-HTᵣₑᵤₚₜₐₖₑ | h | [³H]-paroxetine | **> 1,000** |
| | | | |
| α₁-adrenergic | r | [³H]-prazosin | **> 1,000** |
| α_{1A}-adrenergic | r | [³H]-prazosin | **630** |
| α_{1B}-adrenergic | r | [³H]-prazosin | **> 1,000** |
| α₂-adrenergic | r | [³H]-RX 821002 | **> 1,000** |
| β₁-adrenergic | h | [³H]-CGP 12177 | **50** |
| β₂-adrenergic | h | [³H]-CGP 12177 | **40** |
| β₃-adrenergic | h | [¹²⁵I]-iodocyan opindolol | **> 1,000** |
| NAᵣₑᵤₚₜₐₖₑ | h | [³H]-nisoxetin | **> 1,000** |
| | | | |
| Dopamine-D₁ | h | [³H]-SCH 23390 | **> 1,000** |
| Dopamine-D₂ | h | [³H]-spiperone | **> 1,000** |
| Dopamine-D₃ | h | [³H]-spiperone | **> 1,000** |
| Dopamine-D₄ | h | [³H]-spiperone | **> 1,000** |
| Dopamine-D₅ | h | [³H]-SCH 23390 | **> 1,000** |
| Dopamineᵣₑᵤₚₜₐₖₑ | h | [³H]-GBR 12935 | **> 1,000** |
| | | | |
| Muscarine-M₁ | h | [³H]-pirenzepine | **> 1,000** |
| Muscarine-M₂ | h | [³H]-AFDX-384 | **> 1,000** |
| Muscarine-M₃ | h | [³H]-4-DAMP | **> 1,000** |
| Muscarine-M₄ | h | [³H]-4-DAMP | **> 1,000** |
| Muscarine-M₆ | h | [³H]-4-DAMP | **> 1,000** |
| Histamine-H₁ | h | [³H]-pyrilamine | **> 1,000** |
| Histamine-H₂ | h | [¹²⁵I]-APT | **> 1,000** |
| Histamine-H₃ | r | [³H]-α-methythistamine | **> 10,000** |
| | | | |
| tryptamine | r | [³H]-tryptamine | **> 10,000** |
| melatonin | c | [¹²⁵I]-2-iodomelatonin | **> 10,000** |
| nicotine | r | [³H]-cytisine | **> 10,000** |
| | | | |
| µ-plate | r | [³H]-DAMGO | **> 1,000** |
| κ-opiate | r | [³H]-U 69593 | **> 1,000** |
| δ-opiate | r | [³H]-DPDPE | **> 1,000** |
| nociceptin (ORL₁) | h | [³H]-nociceptine | **> 1,000** |
| sigma | r | [³H]-DTG | **> 1,000** |
| sigma-SG₁ | g | [³H]-pentazocone | **> 1,000** |
| sigma-SG₂ | r | [³H]-DTG | **> 1,000** |
| | | | |
| cannabinoid-CB₁ | h | [³H]-WIN 55,212-2 | **> 10,000** |
| | | | |
| Ca⁺⁺-channel | p | [³H]-fluspirilene | **> 10,000** |
| Ca⁺⁺-channel | r | [³H]-nitrendipine | **> 10,000** |
| Ca⁺⁺-channel | r | [³H]-diltiazem | **> 10,000** |
| Ca⁺⁺-channel | r | [¹²⁵I]-n-conotoxin | **> 1,000** |
| Ca⁺⁺-channel | r | [³H]-D-888 | **130** |
| Ca⁺⁺-channel | r | [³H]-devapamil | **> 10,000** |
| Na⁺-channel | r | [³H]-bathrachotoxinin | **> 10,000** |
| K⁺-channel | r | [¹²⁵I]-α-dendrotoxin | **> 1,000** |
| K⁺-channel | r | [¹²⁰I]-apamin | **> 1,000** |
| | | | |
| Adenosine-A₁ | h | [³H]-DPCPX | **> 1,000** |
| Adenosine-A_{2A} | h | [³H]-CGS 21680 | **> 1,000** |
| Adenosine-A₃ | h | [³H]-AB-MECA | **> 1,000** |
| Purine-P2X | r | [³H]-ab-MeATP | **> 1,000** |
| GABA_{A} | r | [³H]-muscimol | **> 10,000** |
| GABA_{B} | r | [³H]-PK 11195 | **> 1,000** |
| Glycine | r | [³H]-strychnine | **> 10,000** |
| Glycine _{strychn. ins.,} | r | [³H]-MDL 105519 | **> 10,000** |
| NMDA | r | [³H]-CGS 19755 | **> 10,000** |
| | | | |
| angiotensin-AT1 | h | [¹²⁵I]-angiotensin II | **> 1,000** |
| angiotensin-AT2 | h | [¹²⁵I]-CPG 42112A | **> 1,000** |
| benzodiazepine | r | [³H]-diazepam | **> 10,000** |
| bombesin | r | [¹²⁵I]-bombesin | **> 1,000** |
| bradykinin | h | [³H]-bradykinin | **> 1,000** |
| CCK_{A} | h | [³H]-devazepide | **> 1,000** |
| CCK_{B} | h | [³H]-CCK8 | **> 1,000** |
| CCR1 | h | [¹²⁵I]-MIP-1a | **> 1,000** |
| CGRP | h | [¹²⁵I]-CGRPa | **> 1,000** |
| CRF | h | [¹²⁵I]-oCRF | **> 10,000** |
| Endothelin-ET_{A} | h | [¹²⁶I]-endothelin-1 | **> 1,000** |
| Endothelin-ET_{B} | h | [¹²⁵I]-endothelin-1 | **> 1,000** |
| Galanin-GAL₁ | h | [¹²⁵I]-galanin | **> 1,000** |
| Galanin-GAL₂ | h | [¹²⁵I]-galanin | **> 1,000** |
| Interleukine-6 | h | [¹²⁵I]-interleukine-6 | **> 10,000** |
| Interleukine-8 | h | [¹²⁵I]-interleukine-8 | **> 1,000** |
| LTB₄ | g | [³H]-LTB₄ | **> 10,000** |
| LTD₄ | g | [³H]-LTD₄ | **> 10,000** |
| melanocortin | h | [¹²⁵I]-NDP-a-MSH | **> 1,000** |
| | | | |
| Neurokinin-NK₁ | h | [³H]-substance P | **> 1,000** |
| Neurokinin-NK₂ | h | [¹²⁵I]-neurokinin_{A} | **> 1,000** |
| Neurokinin-NK₃ | h | [³H]-SR 142801 | **> 1,000** |
| Neuropeptide Y₁ | h | [¹²⁵I]-PYY | **> 1,000** |
| Neuropeptide Y₂ | h | [¹²⁵I]-PYV | **> 1,000** |
| Neurotensin-NT₁ | h | [¹²⁵I]-neurotensin | **> 1,000** |
| PACAP | r | [¹²⁵I]-PACAP 1-27 | **> 1,000** |
| Prostaglandin-I₂ | h | [³H]-iloprost | **> 1,000** |
| Prostaglandin-H₂ | h | [³H]-SQ 29548 | **> 1,000** |
| somatostatin | m | [¹²⁵I]-somatostatin | **> 1,000** |
| TRH | r | [³H]-TRH | **> 10,000** |
| Tumor necrosis f. | r | [¹²⁵I]-TNFα | **> 1,000** |
| Vasopressine-V_{1A} | h | [³H]-vasopressine | **> 1,000** |
| VIP₁ | h | [¹²⁵I]-VIP | **> 1,000** |

| | | | |
|---|---|---|---|
| ***S¹**: **b** = bovine, **c** = chicken, **g** = guinea pig, **h** = human, **m*** = *mouse, **p** = pig; **r** = rat.* | | | |

## Claims

1. Use of a compound of formula (1): 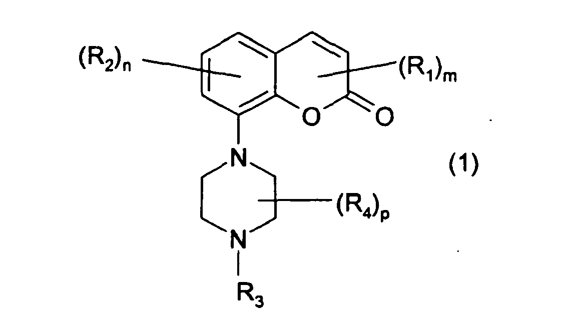 wherein:
R₁ is alkyl(1-4C), alkoxy(1-4C), hydroxyl, alkoxy(1-4C)alkyl(1-4C), pyrrolidinyl, piperidinyl, morpholinyl, halogen, cyano, trifluoromethyl, amino, or mono- or disubstituted amino wherein the substituents are alkyl(1-4C), or alkyl(1-4C) carbonyl,
m has the value 0, 1 or 2,
R₂ is alkyl(1-4C), alkoxy(1-4C), halogen or trifluoromethyl,
n is 0 or 1, on the understanding that (m + n) is at least 1,
R₃ is hydrogen, alkyl(1-3C) or alkenyl(2-3C)
R₄ is alkyl(1-4C), and
p has the value 0, 1 or 2,
and all stereoisomers, and pharmacologically acceptable salts thereof, for the preparation of a pharmaceutical composition for the treatment of pain.

2. Use as claimed in claim 1, wherein said pain is chronic pain including nociceptive, neuropathic, psychogenic pain, and mixed category pain (nociceptive and neuropathic components), including, but not limited to, diabetic neuropathy, neurogenic pain, central pain, somatic pain, visceral and cancer pain, inflammatory pain, post-operative pain, chronic low back pain, sciatica, cervical and lumbar pain, tension headaches, cluster headaches, chronic daily headaches, herpes neuralgia and post-herpetic neuralgia, facial and oral neuralgias and myofascial pain syndromes, phantom limb pain, stump pain and paraplegic pain, dental pain, opioid resistant pain, post-surgical pain including cardic surgery and mastectomy, pain of labour and delivery, post-partum pain, post-stroke pain, angina pain, genitourinary tract pain including pelvic pain and cystitis and vulvar vestibulitis and orchialgia, irritable bowel syndrome, pre-menstrual syndrome pain, pain resulting from burns or chemical injury or sunburn, and bone injury pain.

## Patentansprüche

1. Verwendung einer Verbindung der Formel (1): 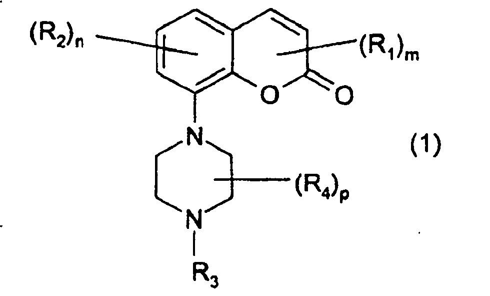 worin:
R₁ für Alkyl(1-4C), Alkoxy(1-4C), Hydroxyl, Alkoxy(1-4C)alkyl(1-4C), Pyrrolidinyl, Piperidinyl, Morpholinyl, Halogen, Cyano, Trifluormethyl, Amino oder mono- oder disubstituiertes Amino steht, wobei die Substituenten Alkyl(1-4C) oder Alkyl(1-4C)carbonyl sind;
m den Wert 0, 1 oder 2 hat,
R₂ für Alkyl(1-4C), Alkoxy(1-4C), Halogen oder Trifluormethyl steht,
n 0 oder 1 ist, unter der Voraussetzung, dass (m + n) mindestens 1 ist,
R₃ für Wasserstoff, Alkyl(1-3C) oder Alkenyl(2-3C) steht,
R₄ für Alkyl(1-4C) steht und
p den Wert 0, 1 oder 2 hat,
und aller Stereoisomere und pharmakologisch annehmbaren Salze davon für die Herstellung einer pharmazeutischen Zusammensetzung zur Behandlung von Schmerzen.

2. Verwendung wie in Anspruch 1 beansprucht, wobei besagter Schmerz chronischer Schmerz ist, einschließlich nozizeptivem, neuropathischem, psychogenem Schmerz und Schmerz von gemischter Kategorie (nozizeptive und neuropatische Bestandteile), einschließlich, aber nicht begrenzt auf diabetische Neuropathie, neurogenen Schmerz, zentralen Schmerz, somatischen Schmerz, Viszeral- und Krebsschmerz, entzündlichen Schmerz, postoperativen Schmerz, chronischen Schmerz im unteren Rückenbereich, Ischialgie, Halswirbel- und Lendenschmerz, Spannungskopfschmerzen, Clusterkopfschmerzen, chronische tägliche Kopfschmerzen, Herpes-Neuralgie und postherpetische Neuralgie, Gesichts- und Mundneuralgien und myofasziale Schmerzsyndrome, Phantomschmerz, Stumpfschmerz und paraplegischen Schmerz, Zahnschmerz, opioidresistenten Schmerz, postoperativen Schmerz einschließlich Herzchirurgie und Mastektomie, Schmerzen von Wehen und Geburt, postpartalen Schmerz, Schmerz nach Schlaganfall, Anginaschmerz, Schmerz des Urogenitaltrakts, einschließlich Unterleibsschmerz und Zystitis und vulväre Vestibulitis und Orchialgie, Reizdarmsyndrom, prämenstruellen Syndromschmerz, Schmerz, welcher sich aus Verbrennungen oder chemischer Verletzung oder Sonnenbrand ergibt, und Knochenverletzungsschmerz.

## Revendications

1. Utilisation d'un composé de formule (1) : 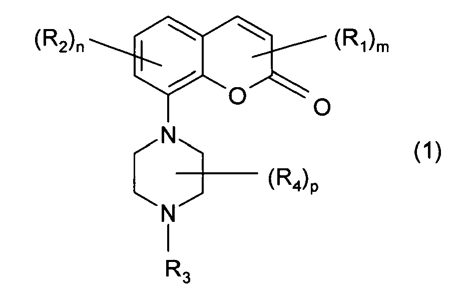 où :
R₁ est alkyle en C1-4, alcoxy en C1-4, hydroxyle, (alcoxy en C1-4)- alkyle en C1-4, pyrrolidinyle, pipéridinyle, morpholinyle, halogène, cyano, trifluorométhyle, amino ou amino mono- ou disubstitué où les substituants sont alkyle en C1-4 ou (alkyl en C1-4)-carbonyle,
m a la valeur 0, 1 ou 2,
R₂ est alkyle en C1-4, alcoxy en C1-4, halogène ou trifluorométhyle,
n est 0 ou 1, étant entendu que (m + n) est au moins égal à 1,
R₃ est hydrogène, alkyle en C1-3 ou alcényle en C2-3
R₄ est alkyle en C1-4 et
P a la valeur 0, 1 ou 2,
et tous ses stéréoisomères et sels pharmaceutiquement acceptables pour la préparation d'une composition pharmaceutique destinée au traitement de la douleur.

2. Utilisation selon la revendication 1, où ladite douleur est une douleur chronique comprenant douleur nociceptive, neuropathique, psychogène et douleur de catégorie mixte (éléments nociceptifs et neuropathiques), comprenant, mais sans y être limitée, neuropathie diabétique, douleur neurogène, douleur centrale, douleur somatique, douleur viscérale et néoplasique, douleur inflammatoire, douleur post-chirurgicale, douleur lombaire chronique, sciatique, douleur cervicale et lombaire, céphalées par tension nerveuse, céphalées vasculaires de Horton, céphalées quotidiennes chroniques, névralgies herpétiques et névralgies post-herpétiques, névralgies faciales et orales et syndromes douloureux myofasciaux, algo-hallucinose, douleurs du moignon et douleur paraplégique, douleur dentaire, douleur résistante aux opioïdes, douleur post-chirurgicale incluant chirurgie cardiaque et mastectomie, douleur du travail et de l'accouchement, douleur du postpartum, douleur post-ictus, douleur angineuse, douleur de l'appareil urogénital comprenant douleur pelvienne et cystite et vestibulite vulvaire et orchialgie, syndrome du côlon irritable, douleur du syndrome pré-menstruel, douleur résultant de brûlures ou de lésion chimique ou d'érythème solaire et douleur de lésion osseuse.
